# EUROPEAN PATENT APPLICATION

(11) **EP 4 714 937 A1**
(43) Date of publication of application: **25.03.2026**
(21) Application number: 24807173.0
(22) Date of filing: 13.05.2024
(51) Int. Cl.: C07C 319/20, C07C 321/14, C08J 11/00, G02B 1/04, G02C 7/00

(54) **METHOD FOR PRODUCING TETRAFUNCTIONAL THIOL WITH EPISULFIDE RESIN AS STARTING MATERIAL**

(30) Priority: 18.05.2023 JP 2023082227
(71) Applicant: MITSUBISHI GAS CHEMICAL COMPANY, INC., Chiyoda-ku Tokyo 100-8324 (JP)
(72) Inventor: TAKEMURA Kouhei, Tokyo 125-8601 (JP); TOMIKAWA Masashi, Tokyo 125-8601 (JP)
(74) Representative: Müller-Boré & Partner Patentanwälte PartG mbB
(86) International application number: PCT/JP2024/017552
(87) International publication number: WO 2024/237221

(57) **Abstract**

According to the present invention, a method for producing a tetrafunctional thiol having a structure represented by the following formula (1), the method including: a step for heating an episulfide resin and a polythiol compound in the presence of a radical initiator, thereby producing the tetrafunctional thiol having a structure represented by the following formula (1) can be provided. 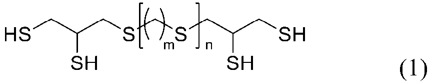 wherein m represents an integer of 0 to 4 and n represents an integer of 0 to 2.

## Description

### Technical Field

The present invention relates to a method for producing a tetrafunctional thiol using an episulfide resin as a starting material.

### Background Art

Plastic lenses are lightweight, exhibit high toughness, and can be easily dyed. The properties particularly required for plastic lenses include low specific gravity, high transparency, low yellowness, optical properties such as a high refractive index and a high Abbe number, as well as high heat resistance and high mechanical strength. A high refractive index enables lens thinning, and a high Abbe number reduces chromatic aberration.

In recent years, numerous sulfur-containing organic compounds have been reported with the aim of achieving a high refractive index and a high Abbe number. In particular, polyepisulfide compounds containing sulfur atoms are known to provide a good balance between refractive index and Abbe number (Patent Literature 1). Furthermore, since polyepisulfide compounds can react with a wide range of compounds, compositions incorporating such compounds have been proposed to improve physical properties. (Patent Literatures 2 to 5).

Optical lenses containing episulfide resin are produced by machining molded articles made of the resin; however, this process generates a large amount of fine machining debris as waste. In addition, defective molded or processed products may be generated during the manufacturing process of molded articles containing episulfide resin.

In the past, such waste products were merely incinerated or buried as industrial waste, raising concerns about their environmental impact. With growing environmental concerns in recent years, there has been a strong demand for technologies that reuse waste as starting materials rather than simply discarding it.

### Citation List

### Patent Literature

Patent Literature 1: Japanese Patent Laid Open No. H09-110979
Patent Literature 2: Japanese Patent Laid Open No. H10-298287
Patent Literature 3: Japanese Patent Laid-Open No. 2001-002783
Patent Literature 4: Japanese Patent Laid-Open No. 2001-131257
Patent Literature 5: Japanese Patent Laid-Open No. 2002-122701

### Summary of Invention

### Technical Problem

An object of the present invention is to provide a technology for effectively utilizing and reusing episulfide resin that has conventionally been treated as industrial waste.

### Solution to Problem

Under such circumstances, the present inventors have conducted intensive studies and found that tetrafunctional thiol can be produced using an episulfide resin as a starting material by the present invention described below, thereby enabling reuse of the episulfide resin that has conventionally been discarded as industrial waste.

Accordingly, the present invention is as follows.
<1> A method for producing a tetrafunctional thiol having a structure represented by the following formula (1), the method comprising: a step for heating an episulfide resin and a polythiol compound in the presence of a radical initiator, thereby producing the tetrafunctional thiol having a structure represented by the following formula (1): 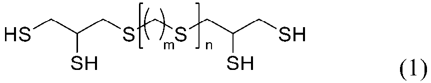 wherein m represents an integer of 0 to 4 and n represents an integer of 0 to 2.
<2> The method for producing a tetrafunctional thiol according to <1> above, wherein the episulfide resin is obtained by polymerizing and curing a composition for an optical material comprising an episulfide compound represented by the following formula (2): 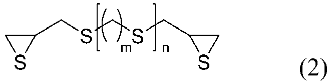 wherein m represents an integer of 0 to 4 and n represents an integer of 0 to 2.
<3> The method for producing a tetrafunctional thiol according to <2> above, wherein the content of the compound represented by the formula (2) in the composition for an optical material is 40 to 99.999 mass%.
<4> The method for producing a tetrafunctional thiol according to <2> or <3> above, wherein the composition for an optical material further comprises a polythiol compound.
<5> The method for producing a tetrafunctional thiol according to any of <2> to <4> above, wherein the composition for an optical material further comprises sulfur.
<6> The method for producing a tetrafunctional thiol according to any of <2> to <5> above, wherein the composition for an optical material further comprises a polyisocyanate compound.
<7> The method for producing a tetrafunctional thiol according to any of <1> to <6> above, wherein the radical initiator is at least one selected from the group consisting of an azo compound, an organic peroxide, and a dihalogen.
<8> The method for producing a tetrafunctional thiol according to any of <1> to <7> above, wherein the polythiol compound is at least one selected from polythiol compounds having a di- or higher functional thiol group.
<9> A composition for an optical material comprising a tetrafunctional thiol obtained by the method for producing a tetrafunctional thiol according to any of <1> to <8> above.
<10> An optical material obtained by polymerizing and curing the composition for an optical material according to <9> above.
<11> An optical lens comprising the optical material according to <10> above.

### Advantageous Effect of Invention

According to the present invention, a tetrafunctional thiol may be produced using, as a starting material, an episulfide resin that has conventionally been discarded as industrial waste. The tetrafunctional thiol produced by the present invention can be reused as a raw material for episulfide resins or as a cleaning agent for tanks used in the production of ophthalmic lenses.

### Description of Embodiments

### [Method for producing tetrafunctional thiol using episulfide resin as starting material]

The method for producing a tetrafunctional thiol having a structure represented by the following formula (1) of the present invention comprises: a step for heating an episulfide resin and a polythiol compound in the presence of a radical initiator, thereby reacting the episulfide resin and the polythiol compound to produce a tetrafunctional thiol.

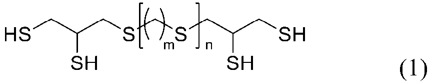

In the formula, m represents an integer of 0 to 4, and preferably an integer of 0 or 1, and n represents an integer of 0 to 2, and preferably an integer of 0 or 1.

According to the method of the present invention, a tetrafunctional thiol may be produced using an episulfide resin as a starting material.

The reason for this effect is presumed to be as follows.

According to the method of the present invention, a polythiol compound reacts with a radical initiator to produce radical active species. These active species subsequently react with an episulfide resin, thereby chemically decomposing the resin. It is presumed that a tetrafunctional thiol is obtained as a decomposition product of the episulfide resin.

It is preferable that the episulfide resin is recovered at any stage in the manufacturing and disposal processes of ophthalmic lenses. As a result, the episulfide resin, which serves as a material for ophthalmic lenses, can be reused.

In the present invention, the episulfide resin generated in at least one of these steps is used as a starting material. The episulfide resin and a polythiol compound are heated in the presence of a radical initiator to induce a reaction between them, thereby yielding a tetrafunctional thiol as a decomposition product of the episulfide resin.

As described above, in the method for producing a tetrafunctional thiol of the present invention, the use of episulfide resin that would otherwise be discarded enables a reduction in the amount of resin incinerated as waste, thereby significantly reducing the environmental impact.

### (Episulfide resin)

The episulfide resin is a starting material in the method of the present invention.

Examples of the episulfide resin include, but are not particularly limited to, the episulfide resins disclosed in known literatures such as Japanese Patent Laid-Open No. 09-110979 and Japanese Patent Laid-Open No. 10-298287.

An episulfide resin obtained by polymerizing and curing a composition for optical materials, which includes a compound represented by the formula (2) as a polymerizable compound, is preferably used as the episulfide resin in the present invention. Specific examples of the compounds of the formula (2) include an episulfide such as bis(β-epithiopropyl)sulfide, bis(β-epithiopropyl)disulfide, bis(β-epithiopropylthio)methane, 1,2-bis(β-epithiopropylthio)ethane, 1,3-bis(β-epithiopropylthio)propane, and 1,4-bis(β-epithiopropylthio)butane. The compound(s) of the formula (2) may be used alone or in combination of two or more.

Among them, bis(β-epithiopropyl)sulfide (n=0 in the formula (2)) and bis(β-epithiopropyl)disulfide (m=0, n=1 in the formula (2)) are preferred, and bis(β-epithiopropyl)sulfide (n=0 in the formula (2)) is most preferred.

The content of the compound represented by the formula (2) in the composition for optical materials is preferably 40 to 99.999 mass%, more preferably 50 to 99.995 mass%, and particularly preferably 60 to 99.99 mass%.

The episulfide resin used in the present invention may include a polythiol compound in the composition for optical materials as a polymerizable compound in order to improve the color tone of the resulting resin when heated. The content of the polythiol compound is usually 1 to 25 mass%, preferably 2 to 25 mass%, and particularly preferably 5 to 20 mass% when the total amount of the composition for optical materials is 100 mass%. When the content of the polythiol compound is less than 1 mass%, yellowing may occur during lens molding, whereas if it exceeds 25 mass%, the heat resistance may deteriorate. The polythiol compound in the present invention may be used alone or in combination of two or more.

Specific examples thereof include methanedithiol, methanetrithiol, 1,2-dimercaptoethane, 1,2-dimercaptopropane, 1,3-dimercaptopropane, 2,2-dimercaptopropane, 1,4-dimercaptobutane, 1,6-dimercaptohexane, bis(2-mercaptoethyl)ether, bis(2-mercaptoethyl)sulfide, 1,2-bis(2-mercaptoethyloxy)ethane, 1,2-bis(2-mercaptoethylthio)ethane, 2,3-dimercapto-1-propanol, 1,3-dimercapto-2-propanol, 1,2,3-trimercaptopropane, 2-mercaptomethyl-1,3-dimercaptopropane, 2-mercaptomethyl-1,4-dimercaptobutane, 2-(2-mercaptoethylthio)-1,3-dimercaptopropane, 4-mercaptomethyl-1,8-dimercapto-3,6-dithiaoctane, 2,4-dimercaptomethyl-1,5-dimercapto-3-thiapentane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 1,1,1-tris(mercaptomethyl)propane, tetrakis(mercaptomethyl)methane, ethylene glycol bis(2-mercaptoacetate), ethylene glycol bis(3-mercaptopropionate), diethylene glycol bis(2-mercaptoacetate), diethylene glycol bis(3-mercaptopropionate), 1,4-butanediol bis(2-mercaptoacetate), 1,4-butanediol bis(3-mercaptopropionate), trimethylolpropane tristhioglycolate, trimethylolpropane trismercaptopropionate, pentaerythritol tetrakisthioglycolate, pentaerythritol tetrakismercaptopropionate, 1,2-dimercaptocyclohexane, 1,3-dimercaptocyclohexane, 1,4-dimercaptocyclohexane, 1,3-bis(mercaptomethyl)cyclohexane, 1,4-bis(mercaptomethyl)cyclohexane, 2,5-dimercaptomethyl-1,4-dithiane, 2,5-dimercaptomethyl-1,4-dithiane, 2,5-bis(2-mercaptoethylthiomethyl)-1,4-dithiane, 2,5-dimercaptomethyl-1-thiane, 2,5-dimercaptoethyl-1-thiane, 2,5-dimercaptomethylthiophene, 1,2-dimercaptobenzene, 1,3-dimercaptobenzene, 1,4-dimercaptobenzene, 1,3-bis(mercaptomethyl)benzene, 1,4-bis(mercaptomethyl)benzene, 2,2'-dimercaptobiphenyl, 4,4'-dimercaptobiphenyl, bis(4-mercaptophenyl)methane, 2,2-bis(4-mercaptophenyl)propane, bis(4-mercaptophenyl)ether, bis(4-mercaptophenyl)sulfide, bis(4-mercaptophenyl)sulfone, bis(4-mercaptomethylphenyl)methane, 2,2-bis(4-mercaptomethylphenyl)propane, bis(4-mercaptomethylphenyl)ether, bis(4-mercaptomethylphenyl)sulfide, 2,5-dimercapto-1,3,4-thiadiazole, 3,4-thiophenedithiol, and 1,1,3,3-tetrakis(mercaptomethylthio)propane.

Among them, bis(2-mercaptoethyl)sulfide, 2,5-dimercaptomethyl-1,4-dithiane, 1,3-bis(mercaptomethyl)benzene, 1,4-bis(mercaptomethyl)benzene, 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 1,1,3,3-tetrakis(mercaptomethylthio)propane, pentaerythritol tetrakismercaptopropionate, pentaerythritol tetrakisthioglycolate, trimethylolpropane tristhioglycolate), and trimethylolpropane trismercaptopropionate are preferred; bis(2-mercaptoethyl)sulfide, 2,5-bis(2-mercaptomethyl)-1,4-dithiane, 4-mercaptomethyl-1,8-dimercapto-3,6-dithiaoctane, 1,3-bis(mercaptomethyl)benzene, pentaerythritol tetrakismercaptopropionate, and pentaerythritol tetrakisthioglycolate are more preferred; and bis(2-mercaptoethyl)sulfide, 2,5-dimercaptomethyl-1,4-dithiane, and 4-mercaptomethyl-1,8-dimercapto-3,6-dithiaoctane are most preferred.

The episulfide resin used in the present invention may include, as a polymerizable compound, a polyisocyanate compound in the composition for optical materials in order to improve the strength of the resulting resin. The content of the polyisocyanate compound is usually 1 to 25 mass%, preferably 2 to 25 mass%, and particularly preferably 5 to 20 mass% when the total amount of the composition for optical materials is 100 mass%. A content of the polyisocyanate compound of less than 1 mass% may cause a reduction in the strength, and a content of more than 25 mass% may result in degradation in the color tone. The polyisocyanate compound used in the present invention may be used alone or in combination of two or more.

Specific examples thereof include diethylene diisocyanate, tetramethylene diisocyanate, hexamethylene diisocyanate, trimethylhexamethylene diisocyanate, cyclohexane diisocyanate, 1,3-bis(isocyanatomethyl)cyclohexane, 1,4-bis(isocyanatomethyl)cyclohexane, isophorone diisocyanate, 2,6-bis(isocyanatomethyl)decahydronaphthalene, lysine triisocyanate, tolylene diisocyanate, o-tolidine diisocyanate, diphenylmethane diisocyanate, diphenyl ether diisocyanate, 3-(2'-isocyanatecyclohexyl)propyl isocyanate, isopropylidene bis(cyclohexylisocyanate), 2,2'-bis(4-isocyanatephenyl)propane, triphenylmethane triisocyanate, bis(diisocyanate tolyl)phenylmethane, 4,4',4"-triisocyanate-2,5-dimethoxyphenylamine, 3,3'-dimethoxybenzidine-4,4'-diisocyanate, 1,3-phenylene diisocyanate, 1,4-phenylene diisocyanate, 4,4'-diisocyanate biphenyl, 4,4'-diisocyanate-3,3'-dimethylbiphenyl, dicyclohexylmethane-4,4'-diisocyanate, 1,1'-methylenebis(4-isocyanatobenzene), 1,1'-methylenebis(3-methyl-4-isocyanatobenzene), m-xylylene diisocyanate, p-xylylene diisocyanate, m-tetramethylxylylene diisocyanate, p-tetramethylxylylene diisocyanate, 1,3-bis(2-isocyanate-2-propyl)benzene, 2,6-bis(isocyanatomethyl)naphthalene, 1,5-naphthalene diisocyanate, bis(isocyanatomethyl)tetrahydrodicyclopentadiene, bis(isocyanatomethyl)dicyclopentadiene, bis(isocyanatomethyl)tetrahydrothiophene, bis(isocyanatomethyl)norbornene, bis(isocyanatomethyl)adamantane, thiodiethyl diisocyanate, thiodipropyl diisocyanate, thiodihexyl diisocyanate, bis[(4-isocyanatomethyl)phenyl]sulfide, 2,5-diisocyanate-1,4-dithiane, 2,5-diisocyanatomethyl-1,4-dithiane, 2,5-diisocyanatomethyl thiophene, dithiodiethyl diisocyanate, and dithiodipropyl diisocyanate.

However, the polyisocyanate compound used in the present invention is not limited to them, and they may be used alone or in combination of two or more.

Among them, preferred examples include at least one compound selected from isophorone diisocyanate, tolylene diisocyanate, diphenylmethane diisocyanate, hexamethylene diisocyanate, m-xylylene diisocyanate, p-xylylene diisocyanate, m-tetramethylxylylene diisocyanate, p-tetramethylxylylene diisocyanate, 1,3-bis(isocyanatomethyl)cyclohexane, 1,4-bis(isocyanatomethyl)cyclohexane, bis(isocyanatomethyl)norbornene, and 2,5-diisocyanatomethyl-1,4-dithiane; in particular, isophorone diisocyanate, tolylene diisocyanate, diphenylmethane diisocyanate, hexamethylene diisocyanate, 1,3-bis(isocyanatomethyl)cyclohexane, and m-xylylene diisocyanate are preferred; and isophorone diisocyanate, m-xylylene diisocyanate, and 1,3-bis(isocyanatomethyl)cyclohexane are most preferred.

The ratio of SH groups in the polythiol compound to NCO groups in the polyisocyanate compound, namely, [the number of SH groups in the polythiol compound/ the number of NCO groups in the polyisocyanate compound], (SH groups/ NCO groups), is preferably 1.0 to 2.5, more preferably 1.25 to 2.25, and further preferably 1.5 to 2.0. If the aforementioned ratio is less than 1.0, lenses may become yellow during molding, whereas if it exceeds 2.5, the heat resistance may deteriorate.

The episulfide resin used in the present invention may include, as a polymerizable compound, sulfur in the composition for optical materials in order to improve the refractive index of the resulting resin. The content of sulfur is usually 0.1 to 15 mass%, preferably 0.2 to 10 mass%, and particularly preferably 0.3 to 5 mass% when the total amount of the composition for optical materials is 100 mass%.

The sulfur used in the present invention may be in any form. Specifically, sulfur may be in the form of powder sulfur, colloidal sulfur, precipitated sulfur, crystalline sulfur, or sublimed sulfur, with powder sulfur, which has fine particles, being preferred.

The sulfur used in the present invention may be produced by any method. Methods for producing sulfur include sublimation purification from natural sulfur ore, extraction by melting underground sulfur deposits, and recovery from hydrogen sulfide derived from the desulfurization of petroleum or natural gas. Any of these methods may be employed.

It is preferable that the sulfur used in the present invention has a particle size smaller than 10 mesh; that is, the sulfur is preferably a fine powder finer than 10 mesh. If the sulfur has a particle size larger than 10 mesh, it may not dissolve completely. The particle size of the sulfur is more preferably smaller than 30 mesh, and most preferably smaller than 60 mesh.

The sulfur used in the present invention has a purity of preferably 98% or more, more preferably 99.0% or more, further preferably 99.5% or more, and most preferably 99.9% or more. When the sulfur has a purity of 98% or higher, the color tone of the resulting optical material is improved compared to that obtained with sulfur with a purity of less than 98%.

### (Radical initiator)

The radical initiator used in the present invention generates radicals that induce the reaction between the episulfide resin and the polythiol compound.

The radical initiator used in the present invention is not particularly limited, but is preferably at least one selected from the group consisting of an azo compound, an organic peroxide, and a dihalogen. More preferably, it is an azo compound due to its ease of handling.

In the present invention, the amount of use of the radical initiator is preferably 1 to 100 parts by mass, more preferably 5 to 50 parts by mass, and most preferably 8 to 25 parts by mass when the amount of use of the episulfide resin is 100 parts by mass.

Specific examples of preferred radical initiators include an azo compound such as 2,2'-azobis(4-methoxy-2,4-dimethylvaleronitrile), 2,2'-azobis(2,4-dimethylvaleronitrile), 2,2'-azobis(isobutyronitrile), 2,2'-azobis(2-methylbutyronitrile), 1,1'-azobis(cyclohexane-1-carbonitrile), 2,2'-azobis[2-(2-imidazolin-2-yl)propane] dihydrochloride, 2,2'-azobis[2-(-imidazolin-2-yl)propane] disulfate dihydrate, 2,2'-azobis[2-(2-imidazolin-2-yl)propane], 2,2'-azobis(2-methylpropionamidine) dihydrochloride, 2,2'-azobis[N-(2-carboxyethyl)-2-methylpropionamidine] n-hydrate, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl 2,2'-azobis(isobutyrate), and 4,4'-azobis(4-cyanovaleric acid), and an organic peroxide such as di-tert-butyl peroxide, dibenzoyl peroxide, and dicumyl peroxide. Among them, 2,2'-azobis(isobutyronitrile) (AIBN) is more preferred.

### (Polythiol compound)

The polythiol compound functions as a reactant in the reaction with the episulfide resin.

The polythiol compound used in the present invention may be used alone or in combination of two or more.

In the present invention, the amount of use of polythiol is preferably 100 to 5,000 parts by mass, more preferably 200 to 2,000 parts by mass, and most preferably 500 to 1,500 parts by mass when the amount of use of the episulfide resin is 100 parts by mass.

Specific examples of the polythiol compound used in the present invention include methanedithiol, methanetrithiol, 1,2-dimercaptoethane, 1,2-dimercaptopropane, 1,3-dimercaptopropane, 2,2-dimercaptopropane, 1,4-dimercaptobutane, 1,6-dimercaptohexane, bis(2-mercaptoethyl)ether, bis(2-mercaptoethyl)sulfide, 1,2-bis(2-mercaptoethyloxy)ethane, 1,2-bis(2-mercaptoethylthio)ethane, 2,3-dimercapto-1-propanol, 1,3-dimercapto-2-propanol, 1,2,3-trimercaptopropane, 2-mercaptomethyl-1,3-dimercaptopropane, 2-mercaptomethyl-1,4-dimercaptobutane, 2-(2-mercaptoethylthio)-1,3-dimercaptopropane, 4-mercaptomethyl-1,8-dimercapto-3,6-dithiaoctane, 2,4-dimercaptomethyl-1,5-dimercapto-3-thiapentane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 1,1,1-tris(mercaptomethyl)propane, tetrakis(mercaptomethyl)methane, ethylene glycol bis(2-mercaptoacetate), ethylene glycol bis(3-mercaptopropionate), diethylene glycol bis(2-mercaptoacetate), diethylene glycol bis(3-mercaptopropionate), 1,4-butanediol bis(2-mercaptoacetate), 1,4-butanediol bis(3-mercaptopropionate), trimethylolpropane tristhioglycolate, trimethylolpropane trismercaptopropionate, pentaerythritol tetrakisthioglycolate, pentaerythritol tetrakismercaptopropionate, 1,2-dimercaptocyclohexane, 1,3-dimercaptocyclohexane, 1,4-dimercaptocyclohexane, 1,3-bis(mercaptomethyl)cyclohexane, 1,4-bis(mercaptomethyl)cyclohexane, 2,5-dimercaptomethyl-1,4-dithiane, 2,5-dimercaptomethyl-1,4-dithiane, 2,5-bis(2-mercaptoethylthiomethyl)-1,4-dithiane, 2,5-dimercaptomethyl-1-thiane, 2,5-dimercaptoethyl-1-thiane, 2,5-dimercaptomethylthiophene, 1,2-dimercaptobenzene, 1,3-dimercaptobenzene, 1,4-dimercaptobenzene, 1,3-bis(mercaptomethyl)benzene, 1,4-bis(mercaptomethyl)benzene, 2,2'-dimercaptobiphenyl, 4, 4'-dimercaptobiphenyl, bis(4-mercaptophenyl)methane, 2,2-bis(4-mercaptophenyl)propane, bis(4-mercaptophenyl)ether, bis(4-mercaptophenyl)sulfide, bis(4-mercaptophenyl)sulfone, bis(4-mercaptomethylphenyl)methane, 2,2-bis(4-mercaptomethylphenyl)propane, bis(4-mercaptomethylphenyl)ether, bis(4-mercaptomethylphenyl)sulfide, 2,5-dimercapto-1,3,4-thiadiazole, 3,4-thiophenedithiol, and 1,1,3,3-tetrakis(mercaptomethylthio)propane.

Among them, preferred examples include bis(2-mercaptoethyl)sulfide, 2,5-dimercaptomethyl-1,4-dithiane, 1,3-bis(mercaptomethyl)benzene, 1,4-bis(mercaptomethyl)benzene, 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 1,1,3,3-tetrakis(mercaptomethylthio)propane, pentaerythritol tetrakismercaptopropionate, pentaerythritol tetrakisthioglycolate, trimethylolpropane tristhioglycolate, and trimethylolpropane trismercaptopropionate; bis(2-mercaptoethyl)sulfide, 2,5-bis(2-mercaptomethyl)-1,4-dithiane, 4-mercaptomethyl-1,8-dimercapto-3,6-dithiaoctane, 1,3-bis(mercaptomethyl)benzene, pentaerythritol tetrakismercaptopropionate, and pentaerythritol tetrakisthioglycolate are more preferred; and bis(2-mercaptoethyl)sulfide, 2,5-dimercaptomethyl-1,4-dithiane, 4-mercaptomethyl-1,8-dimercapto-3,6-dithiaoctane are most preferred.

The application of the tetrafunctional polythiol as the intended product is not particularly limited. As the intended product, the tetrafunctional thiol may be used, for example, in the production of episulfide resin.

Specific examples of applications of the tetrafunctional thiol as the intended product include its use in the production of optical materials (e.g., ophthalmic lenses). Furthermore, the tetrafunctional thiol may be used as a cleaning agent for tanks used in the production of ophthalmic lenses.

### (Method for producing tetrafunctional thiol)

The method for producing a tetrafunctional thiol of the present invention comprises a step for heating an episulfide resin and a polythiol compound in the presence of a radical initiator.

The following is a production example: an episulfide resin and a polythiol compound are mixed, and a radical initiator is added thereto, and the mixture is stirred at preferably 80 to 120°C (more preferably 90 to 110°C) for preferably 2 to 10 (more preferably 4 to 8) hours. The resulting liquid is extracted with ethyl acetate and water, and the organic layer is then concentrated. The concentrated liquid is passed through a column to produce a tetrafunctional thiol having a structure represented by the above formula (1).

### Examples

In the following, the present invention will be described with reference to Examples and a Comparative Example, but the present invention is not limited to the following Examples.

An episulfide resin recovered at any stage in the manufacturing process of ophthalmic lenses, or the manufacturing and disposal processes of eyeglasses may be used. Alternatively, an episulfide resin independently prepared may be used.

### <Preparation of episulfide resin powder A>

Step (1): 68 parts by mass of bis(β-epithiopropyl)sulfide represented by the following structural formula, 25 parts by mass of sulfur, and 1.3 parts by mass of 2-(2-hydroxy-5-t-octylphenyl)-2H-benzotriazole as an ultraviolet absorber were added to a reactor, and the mixture was stirred at 60°C until all components were dissolved. Subsequently, 0.5 parts by mass of 2-mercapto-1-methylimidazole was added as a preliminary reaction catalyst, and the mixture was allowed to undergo a preliminary reaction at 60°C for 50 minutes, and then cooled to 20 °C. The resulting reaction solution is referred to as preliminary reaction solution A.

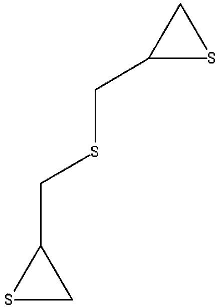

Step (2): Separately from Step (1) above, 7 parts by mass of bis(2-mercaptoethyl)sulfide represented by the following structural formula, 0.2 parts by mass of dibutyltin dichloride as a reaction regulator, and 0.03 parts by mass of triethylbenzylammonium chloride as a curing catalyst were mixed at 20°C, thereby preparing a homogeneous solution A.

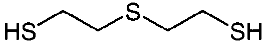

Step (3): 7 parts by mass of the homogeneous solution A prepared in Step (2) above was added to and mixed with 100 parts by mass of the preliminary reaction solution A prepared in Step (1) above, and defoaming was performed at 20°C under a reduced pressure of 20 Torr for 80 minutes, thereby preparing a composition for optical materials A.

Step (4): The composition for optical materials A obtained in Step (3) above was filtered through a PTFE (polytetrafluoroethylene) filter having a pore size of 1 µm and simultaneously poured into a lens mold, which was composed of two glass plates and tape and had a mold diameter of 75 mm and a central thickness of 5 mm. After pouring, the composition for optical materials A in the mold was gradually heated from 10°C to 60°C over 30 hours, held at 60°C for 7 hours, further heated to 100°C over 4 hours, and then held at 100°C for 4 hours, thereby undergoing polymerization and curing. After cooling, the composition was removed from the mold to obtain a cured optical material. The resulting optical material was ground using a grinder, yielding episulfide resin powder A.

### <Preparation of episulfide resin powder B>

Step (1): 79 parts by mass of bis(β-epithiopropyl)sulfide represented by the above structural formula, 14 parts by mass of sulfur, and 1.3 parts by mass of 2-(2-hydroxy-5-t-octylphenyl)-2H-benzotriazole as an ultraviolet absorber were added to a reactor, and the mixture was stirred at 60°C until all components were dissolved. Subsequently, 0.5 parts by mass of 2-mercapto-1-methylimidazole was added as a preliminary reaction catalyst, and the mixture was allowed to undergo a preliminary reaction at 60°C for 50 minutes, and then cooled to 20°C. The resulting reaction solution is referred to as preliminary reaction solution B.

Step (2): Separately from Step (1) above, 7 parts by mass of bis(2-mercaptoethyl)sulfide represented by the above structural formula, 0.2 parts by mass of dibutyltin dichloride as a reaction regulator, and 0.03 parts by mass of triethylbenzylammonium chloride as a curing catalyst were mixed at 20°C, thereby preparing a homogeneous solution B.

Step (3): 7 parts by mass of the homogeneous solution B prepared in Step (2) above was added to and mixed with 100 parts by mass of the preliminary reaction solution B prepared in Step (1) above, and defoaming was performed at 20°C under a reduced pressure of 20 Torr for 80 minutes, thereby preparing a composition for optical materials B.

Step (4): The composition for optical materials B obtained in Step (3) above was filtered through a PTFE (polytetrafluoroethylene) filter having a pore size of 1 µm and simultaneously poured into a lens mold, which was composed of two glass plates and tape and had a mold diameter of 75 mm and a central thickness of 5 mm. After pouring, the composition for optical materials B in the mold was gradually heated from 10°C to 60°C over 30 hours, held at 60°C for 7 hours, further heated to 100°C over 4 hours, and then held at 100°C for 4 hours, thereby undergoing polymerization and curing. After cooling, the composition was removed from the mold to obtain a cured optical material. The resulting optical material was ground using a grinder, yielding episulfide resin powder B.

### (Example 1)

1 g of the episulfide resin powder A obtained as described above was mixed with 10 g of bis(2-mercaptoethyl)sulfide represented by the above structural formula, and 0.1 g of azodiisobutyronitrile AIBN was added thereto as a radical initiator, and the mixture was stirred at 100°C for 5 hours. The resulting transparent liquid was extracted with ethyl acetate and water, and the organic layer was concentrated. The concentrated liquid was passed through a column to produce 433 mg of bis(2,3-dimercaptopropyl)sulfide represented by the following structural formula. The NMR data of the obtained compound, measured using deuterochloroform, are shown below.

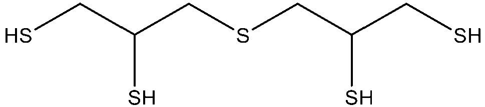

¹H-NMR (500 MHz, CHLOROFORM-D) δ3.14-3.11(2H), 2.97-2.87 (8H), 2.07-2.04 (2H), 1.66-1.625 (2H)

### (Example 2)

1 g of the episulfide resin powder B obtained as described above was mixed with 10 g of bis(2-mercaptoethyl)sulfide represented by the above structural formula, and 0.1 g of azodiisobutyronitrile AIBN was added thereto as a radical initiator, and the mixture was stirred at 100°C for 5 hours. The resulting transparent liquid was extracted with ethyl acetate and water, and the organic layer was concentrated. The concentrated liquid was passed through a column to produce 433 mg of bis(2,3-dimercaptopropyl)sulfide represented by the above structural formula.

### (Comparative Example 1)

1 g of the episulfide resin powder A obtained as described above was mixed with 10 g of ethylene glycol, and 0.1 g of azodiisobutyronitrile AIBN was added thereto as a radical initiator. The mixture was stirred at 100 °C for 5 hours; however, no reaction occurred, and the episulfide resin powder was recovered as is.

**[Table 1]**

| | | Example 1 | Example 2 | Comparative Example 1 |
|---|---|---|---|---|
| Episulfide resin | Type | A | B | A |
| | Amount (g) | 1 | 1 | 1 |
| Polythiol compound | Type | bis(2-mercaptoethyl)sulfide | bis(2-mercaptoethyl)sulfide | bis(2-mercaptoethyl)sulfide |
| | Amount (g) | 10 | 10 | 10 |
| Radical initiator AIBN | Amount (g) | 0.1 | 0.1 | 0.1 |
| Form of episulfide resin after reaction | | Transparent liquid | Transparent liquid | Remained undissolved in powder form |
| Tetrafunctional thiol obtained | | bis(2,3-dimercaptopropyl)sulfide | bis(2,3-dimercaptopropyl)sulfide | - |

As shown in Table 1, in Examples 1 and 2, in which the episulfide resin and the polythiol compound were heated in the presence of a radical initiator to react the episulfide resin with the polythiol compound, high purity tetrafunctional thiol, i.e., bis(2,3-dimercaptopropyl)sulfide, was obtained.

In contrast, in Comparative Example 1 in which the episulfide resin and ethylene glycol were reacted, no decomposition reaction occurred.

## Claims

1. A method for producing a tetrafunctional thiol having a structure represented by the following formula (1), the method comprising: a step for heating an episulfide resin and a polythiol compound in the presence of a radical initiator, thereby producing the tetrafunctional thiol having a structure represented by the following formula (1): 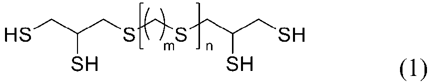 wherein m represents an integer of 0 to 4 and n represents an integer of 0 to 2.

2. The method for producing a tetrafunctional thiol according to claim 1,
wherein the episulfide resin is obtained by polymerizing and curing a composition for an optical material comprising an episulfide compound represented by the following formula (2): 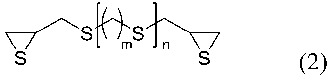 wherein m represents an integer of 0 to 4 and n represents an integer of 0 to 2.

3. The method for producing a tetrafunctional thiol according to claim 2,
wherein a content of the compound represented by the formula (2) in the composition for an optical material is 40 to 99.999 mass%.

4. The method for producing a tetrafunctional thiol according to claim 2 or 3, wherein the composition for an optical material further comprises a polythiol compound.

5. The method for producing a tetrafunctional thiol according to any of claims 2 to 4, wherein the composition for an optical material further comprises sulfur.

6. The method for producing a tetrafunctional thiol according to any of claims 2 to 5, wherein the composition for an optical material further comprises a polyisocyanate compound.

7. The method for producing a tetrafunctional thiol according to any of claims 1 to 6, wherein the radical initiator is at least one selected from the group consisting of an azo compound, an organic peroxide, and a dihalogen.

8. The method for producing a tetrafunctional thiol according to any of claims 1 to 7, wherein the polythiol compound is at least one selected from polythiol compounds having a di- or higher functional thiol group.

9. A composition for an optical material comprising a tetrafunctional thiol obtained by the method for producing a tetrafunctional thiol according to any of claims 1 to 8.

10. An optical material obtained by polymerizing and curing the composition for an optical material according to claim 9.

11. An optical lens comprising the optical material according to claim 10.
